# EUROPEAN PATENT APPLICATION

(11) **EP 0 958 820 A1**
(43) Date of publication of application: **24.11.1999**
(21) Application number: 98401197.3
(22) Date of filing: 19.05.1998
(51) Int. Cl.: A61K 31/505

(54) **Use of an imidazol derivative for the manufacture of a medicament for treating auto-immunodiseases**

(71) Applicant: Sanofi-Synthélabo, 75013 Paris (FR)
(72) Inventor: Funayama, Kaori c/o Mitsubishi Chem. Corp., Yokohama-shi, Kanagawa 227-8502 (JP); Honda Kumiko, c/o Mitsubishi Chem. Corp., Yokohama-shi, Kanagawa 227-8502 (JP); Yamada, Noboru, c/o Mitsubishi Chem. Corp., Yokohama-shi, Kanagawa 227-8502 (JP)
(74) Representative: Thouret-Lemaitre, Elisabeth

(57) **Abstract**

Use of a compound represented by the following general formula (I) 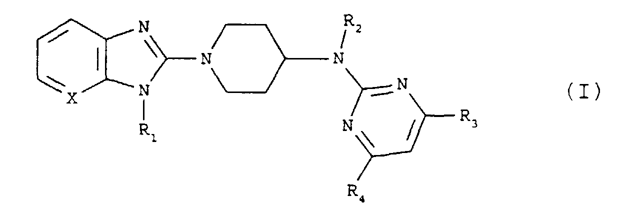 where X is a group -CH- or a nitrogen atom ;
R₁ is a hydrogen atom, a non-substituted benzyl group, a substituted benzyl group ; a non-substituted heterocyclic methyl group wherein the heterocyclic part is a pyridyl, thienyl or furyl ; or a substituted heterocyclic methyl group ;
R₂ is a hydrogen atom or (C₁₋₄) alkyl group ;
R₃ is a hydrogen atom or hydroxy group ;
R₄ is a hydrogen atom or (C₁₋₄) alkyl group, its tautomer when R₃ is an hydroxy group, their pharmaceutically permissible acid addition salts, or any of their hydrates for the manufacture of a medicament for treating auto-immunodiseases.

## Description

The present invention relates to the use of an imidazol derivative for the manufacture of a medicament for treating auto-immunodiseases.

The European Patent EP 0 217 700 describes imidazol derivatives which have a histamine antagonistic action and can be used for therapy of various allergic symptoms such as respiratory allergy, cutaneous allergy and ocular allergy, and that these compounds have no anticholin and antiserotonin actions. A compound described in the European Patent EP 0 217 700, is 2-([1-{1-[(4-fluorophenyl)methyl]-1*H*-benzimidazol-2-yl}-4-piperidyl]methylamino)-4-pyrimidinol known as mizolastine.

The European patent EP 0 834 315 relates to the use of Mizolastine in the treatment of rhinitis.

However, it has not been reported, so far as the present inventors know, that these compounds can be used for treating auto-immunodiseases nor that they have an inhibitory action on production of cytokines IL-4 (interleukin-4) and TNF-α (tumour necrosis factor-α).

The object of the present invention is to provide a new therapeutic agent for treating auto-immunodiseases.

The therapeutic agent of the present invention contains an imidazol derivative represented by the following general formula (I) 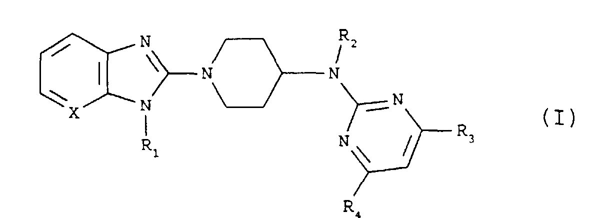 where X is a group -CH- or a nitrogen atom ;
R₁ is a hydrogen atom, a non-substituted benzyl group, a benzyl group substituted by up to three substitutional groups selected from the group consisting of halogen, trifluoromethyl, (C₁₋₄) alkyl, (C₁₋₄) alkoxy, cyano, methylthio, methylsulfinyl or methylsulfonyl groups ; a non-substituted heterocyclic methyl group wherein the heterocyclic part is a pyridyl, thienyl or furyl ; or a heterocyclic methyl group wherein the heterocyclic part is substituted by one or more substitutional groups selected from the group consisting of halogen, trifluoromethyl, (C₁₋₄) alkyl, (C₁₋₄) alkoxy, cyano, methylthio, methylsulfinyl or methylsulfonyl groups ;
R₂ is a hydrogen atom or (C₁₋₄) alkyl group ;
R₃ is a hydrogen atom or hydroxy group ;
R₄ is a hydrogen atom or (C₁₋₄) alkyl group.

The free form of compounds described above, their pharmaceutically permissible acid addition salts, or any of their hydrates can be used as an effective ingredient of the therapeutic agent of the present invention.

Some examples for the pharmaceutically permissible acid addition salts are inorganic acid salts such as hydrochlorides, hydrobromides, sulfates, phosphates and nitrates ; and organic acid salts such as acetates, succinates, adipates, propionates, tartrates, fumarates, maleates, oxalates, citrates, benzoates, toluenesulfonates and methanesulfonates.

A preferable compound of the formula (I) above is a compound whose X is a group -CH- or nitrogen atom, R₁ is 4-fluorobenzyl group, and R₂, R₃ and R₄ are compounds which have the same significance as above.

Among the compounds of the formula (I) whose X is a group -CH-, preferable compounds are those whose R₁ is a benzyl group which has one or two substitutional groups, particularly one substitutional group at the 4-site which is a fluorine or chlorine atom or a methyl, methoxy, methylthio, trifluoromethyl, cyano or methylsulfinyl group.

If R₃ is a hydroxy group and R₄ is a hydrogen atom, these compounds can exist in tautomeric forms which are part of the invention.

Concrete examples for preferable compounds among these ones are those described in European Patent No. EP 0 217 700 and the most preferable one is 2-([1-{1-[(4-fluorophenyl)methyl]-1*H*-benzimidazol-2-yl}-4-piperidyl]methylamino)-4-pyrimidinol (herein below sometimes referred to as "mizolastine" or "MKC-431").

The compound represented by the above general formula (I) which is an effective ingredient of the therapeutic agent of the present invention described above is a known compound described in European Patent EP 0 217 700, and it can readily be manufactured in accordance with the description of the application.

The above-mentioned compound itself which is an effective ingredient may be used as the therapeutic agent of the present invention, but it is preferable to use medical composites comprising the above effective ingredient manufactured by using common additives for pharmaceutical preparations. Some examples for such medical composites are tablets, capsules, granules, balls or pills, troches, liquid drugs, injections, suppositories, ointments and poultices, which are orally (including sublingual administration) or non-orally administered.

The medical composite for oral use can be manufactured by conventional all-purpose methods such as mixing, packing or tablet making. The effective ingredient may be distributed in a medical composite using a large amount of filler by using a repetitive blending. For example, a tablet or capsule used for the oral administration is preferably provided as a unit dose which may contain a carrier, generally used for pharmaceutical preparation, such as a binder, filler, diluent, tableting agent, lubricant, disintegrator, colouring agent, flavouring agent and infiltrating agent. Tablets may be prepared following methods known in the art, for example, they may be coated tablets using a coating agent.

Some examples for a preferable filler are cellulose, mannitol and lactose, and starch and polyvinyl pyrrolidone which are disintegrators, starch derivatives such as sodium starch glycolate and dodecyl sodium sulfate which is a lubricant may be used as additives for the pharmaceutical preparation. The medical composites for oral administration in the form of liquid drugs are provided, for example, as aqueous or oily suspension, solution, emulsion, medical composites such as syrup or elixir, or dry medical composites redissolved with water or other suitable media before use.

To such liquid drugs, general additives, e.g. suspending agent such as sorbitol, syrup, methylcellulose, gelatin, hydroxyethylcellulose, carboxymethylcellulose, aluminium stearate gel or hydrogenated edible fat ; emulsifying agents such as lecithin, sorbitan monooleate and gum arabic ; oily esters such as almond oil, rectified coconut oil, glycerin ester ; non-aqueous solvents such as propylene glycol and ethyl alcohol (also includable of edible oil) ; preservatives such as methyl ester, ethyl ester or propyl ester of p-hydroxybenzoic acid or sorbic acid, and common flavouring agents or colouring agents can be blended where necessary.

As the medical composites suitable for non-oral administration, liquid medical composites or medical composites in a form of suppository or cataplasm which contain above-mentioned effective ingredient and sterilized media can be manufactured. For example, the effective ingredient may be suspended, dissolved or emulsified depending on the medium and concentration, e.g. the solution composites for non-oral use can preferably be manufactured by dissolving the effective ingredient in a medium and filtering the solution to sterilize, then filling it into suitable vials or ampules and sealing. Water may be removed by freeze drying after preparing the aqueous solution composite for increasing the stability.

Suspensions for non-oral use are manufactured by substantially the same method as that for the solution composites for non-oral use described above. For example, they are manufactured by suspending the effective ingredient in a medium, sterilizing using ethylene oxide, etc., and further suspending in a sterilized medium. Surfactants or lubricants may be added, where necessary, on manufacturing the suspensions, etc. to make the effective ingredient uniformly distributed in the pharmaceutical preparations. The medical composites in other forms can also be manufactured by methods known to those skilled in the art, and the form and method for manufacturing of the medical composite which is one aspect of the drug of the present invention are not limited to those mentioned above.

The above-mentioned medical composites for oral use, e.g. tablets, capsules and granules generally contain 0.1 - 95 percentage by weight, preferably 0.5 - 10 percentage by weight of the effective ingredient, while the medical composites for non-oral use, e.g. injections generally contain 0.05 - 40 percent by weight, preferably 0.1 - 20 percent by weight of the effective ingredient.

The dose of the therapeutic agent of the present invention may be fitly decided in accordance with the age, condition of health, weight, severity of disease, sorts and frequency of the therapy or treatment simultaneously performed, and nature of desired effect, etc. of patients. Generally, one dose for an adult may be 0.01 - 100 mg/kg-weight, preferably 0.1 - 10 mg/kg-weight as the amount of the effective ingredient and the drug may be administered once to a few times daily.

The present invention will be particularly described with embodiments, which the present invention is not limited to.

Mizolastine, used in the embodiments below, (2-(1-{1-[(4-fluorophenyl)methyl]-1*H*-benzimidazol-2-yl}-4-piperidyl]methylamnino)-4-pyrimidinol) was prepared in accordance with the method described in the European Patent No. 0 217 700.

### Example 1 :

**1. Test drugs**
   Mizolastine and terfenadine used were synthesised according to well known method in the art. Ketotifen fumarate (hereinafter 'ketotifen') was obtained from Toa Boshoku and dexamethasone from Sigma.
**2. Animals**
   CEA/J female mice and Balb/c female mice were used.
**3. Preparation of bone marrow-derived mast cells (hereinafter 'BMMC')**
   Mouse BMMC were prepared in accordance with the method of Saito et al. J. Immunol. 138: 3927-3934, 1987. That is to say, femoral bone marrow cells from female CBA/J mice were suspended to concentrations of 2-5 x 10⁵/ml in RPMI 1640 medium to which 50% of WEHI-3 culture supernatant had been added (containing 10% FBS, 5 x 10⁻⁵M 2-mercaptoethanol, 50IU/ml penicillin, 50 g/ml streptomycin).
**4. Production of IL-4 and TNF-α from mouse BMMC**
   A 1/250 volume of anti-mouse DNP-IgE monoclonal antibody (Seikagaku Kogyo, 25 g/ml) was added to mouse BMMC (2 x 10⁶ cells/ml) and they were further sensitised by being incubated overnight. The sensitised BMMC were washed three times with RPMI 1640 medium, then suspended in RPMI 1640 medium to which 50% of WEHI-3 culture supernatant had been added to give 1.25 x 10⁶ cells/ml (IL-4) or 3.75 x 10⁶ cells/ml (TNF-α ). Having added 40 l of a test drug solution prepared as a ten-fold dilution to 320 l/well of the cell suspension and treated the cells for 30 minutes to one hour at 37°C in 5% CO₂, antigen stimulation was applied adding 40 l of a solution of dinitrophenyl human serum albumin (Sigma; hereinafter 'DNP-HSA'; 300ng/ml) (reaction solution 400 l/well/48 well plate, final cell concentration 1∼3 x 10⁶ cells/ml, final antigen concentration 30ng/ml). The level of the cytokine in the supernatant fluid from the culture was assayed by ELISA 18 hours (IL-4) or 4 hours (TNF-α ) following stimulation with the antigen.
**5. TNF-α production from mouse macrophages** (according to the method described by Tebo, J.M., Hamilton, T.A. in Cellular Immunology 153: 479-491, 1994)
   Having dissolved glycogen (Wako Junyaku) in PBS to 5% (w/v) and sterilised the solution by autoclaving, 1ml/mouse thereof was administered into the peritoneal cavity of female Balb/c mice. The mice were killed by dislocating the cervical vertebrae four days later, 12ml of TS2 medium then being infused into the peritoneal cavity and recovered to collect the peritoneal cells. After being centrifuged (1000rpm, 4°C, 5 minutes) and washed, they were suspended in TS2 medium (+10% FCS) and cultured for 2-3 hours at 10⁵ cells/well (37°C, 5% CO₂, 48 well plate) to make the macrophages adhere to the well. Having discarded the supernatant fluid, the individual wells were washed once with 500 l of TS2 medium to remove free cells. TS2 medium (+10% FCS) and a test drug solution were added to the macrophages adhering to each well and okadaic acid (L.C. Laboratories) was added 30 minutes later (final concentration 0.2µM, 400 l/well). The level of TNF-α in the culture supernatant fluid was determined by ELISA 18 hours after stimulating with okadaic acid.
**6. Assay of IL-4**
   IL-4 was assayed in accordance with the protocol for the ELISA kit (Endogen). That is to say, 50 l of plate reagent were added to each well, then 50 l/well of a standard or two-fold diluted sample were also added and allowed to react for two hours at 37°C. Having washed with a wash buffer, 100 l/well of anti-mouse IL-4 conjugate was added and reacted for one hour at 37°C. Following washing with a wash buffer, 100 l/well of a TMB substrate solution were added and colouration was permitted for 30 minutes at room temperature whilst shading from light. 100 l/well of a stop buffer were added and measurements were then taken at the two wavelengths of 450nm and 550nm using an immunoreader (Wako Junyaku, THERMOmax).
**7. Assay of TNF-α**
   TNF-α was assayed by sandwich ELISA. That is, 50 l/well of mouse anti-TNF-α monoclonal antibody (R & D Systems, 25 g/ml) were added to a 96 well plate, coated overnight at 4°C and blocked with 200 l/well of 0.5% BSA/PBS.Az⁺. 50 l/well of the standard and each sample were reacted for two hours at room temperature. Next, 50 l/well of a biotinated mouse anti-TNF-α monoclonal antibody were reacted at room temperature for two hours, then 50 l/well of peroxidase-conjugated avidin (Capel, x1000 dilution) were reacted for one hour at room temperature. Colouring was undertaken using a peroxidase colouring kit (Sumitomo Bakelite, substrate TMBZ) for 30 minutes whereupon measurements were taken with an immunoreader (Wako Junyaku, THERMOmax) at a wavelength of 450nm. The plate was washed at each stage with PBS-Tween 20.
**8. Statistical analysis**
   The results were expressed as mean values S.E. Significant difference was tested by the Dunnett method between the control group and each test drug group following one-way variance analysis. IC₅₀ values were calculated from the dose-response curve.

### II. RESULTS

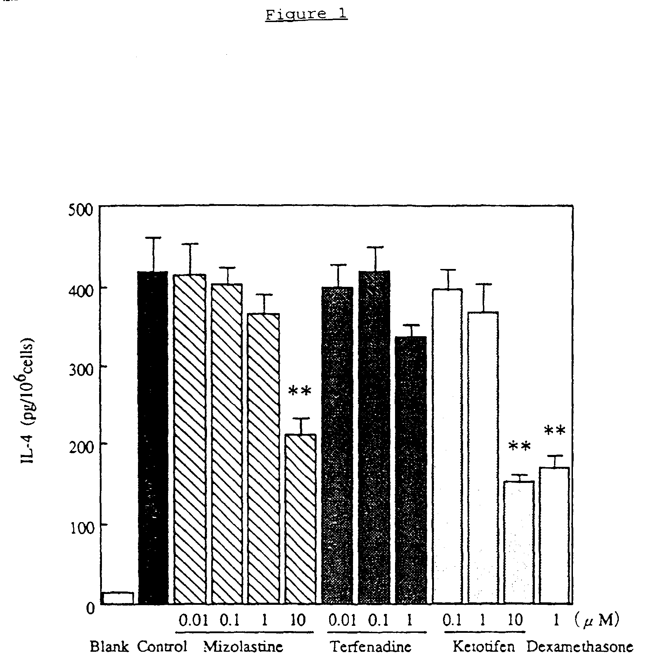

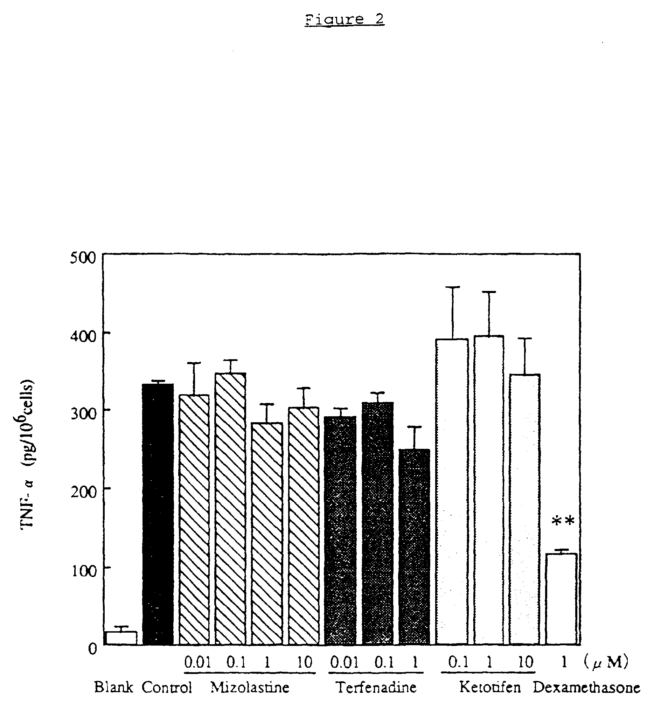

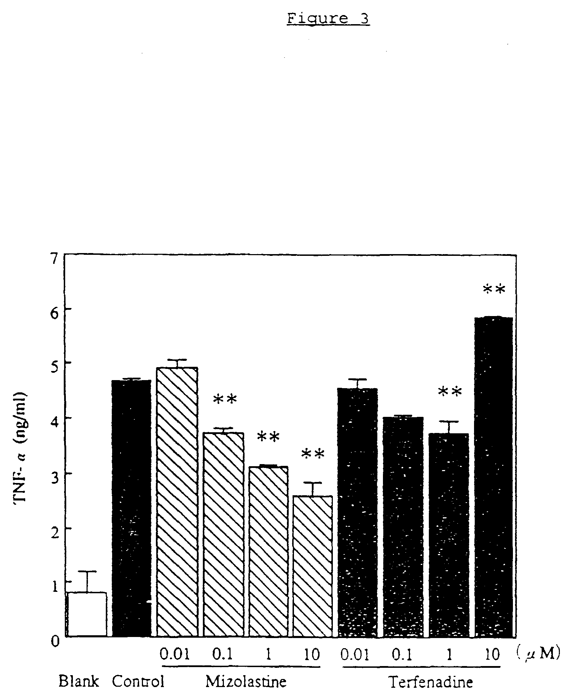

**1. IL-4 and TNF-α production from mouse BMMC**
   Upon adding DNP-HSA to mouse BMMC sensitised with anti-DNP-IgE, IL-4 production was observed at 405pg/10⁶ cells and TNF-α production at 315pg/10⁶ cells.
   Mizolastine investigated 0.01-10 µM showed 50% inhibition of IL-4 production at 10 µM (Fig. 1). The control drug terfenadine showed no activity at concentrations of 0.01∼1 µM. Ketotifen showed inhibitory activity of 65.6% at a concentration of 10 µM. Dexamethasone, on the other hand, showed inhibitory activity of 61.6% at a concentration of 1 µM.
   Mizolastine was not active against TNF-α production at 0.01∼10 µM (Fig. 2). The control drugs terfenadine (0.01∼1 µM) and ketotifen (0.1∼10 µM) also showed no activity. Dexamethasone, on the other hand, showed inhibitory activity of 68.7% at a concentration of 1 µM.
   As terfenadine manifested cytotoxic effects at a concentration of 10 µM (confirmed by release of LDH: lactate dehydrogenase, from the cells), no investigation was possible at concentrations higher than this.
**2. TNF-α production from mouse macrophages**
   Mizolastine in a concentration range provided concentration-dependent inhibition of TNF-α production from mouse peritoneal macrophages induced by glycogen, with IC₅₀ of 4.1 µM (Fig. 3). The control drug terfenadine showed 24.6% inhibition at a concentration of 1 µM.

As the results of tests Mizolastine showed inhibitory activity against the production of IL-4 from mouse BMMC and against the production of TNF-α from mouse macrophages (IC₅₀ = 4.1 µM), mizolastine did not appear to affect the production of TNF-α from mouse BMMC.

The control drug terfenadine had no effect on the production of IL-4 and the production of TNF-α from mouse BMMC at concentrations of 0.01-1 µM and no investigation could be conducted at 10 µM due to cell ageing. On the other hand, 24.6% inhibition of TNF-α production from mouse macrophages was found at a concentration of 1 µM. It was thus thought that terfenadine had not affected the production of these cytokines at the concentrations which could be investigated. Ketotifen, like mizolastine, inhibited IL-4 production from mouse BMMC but had no effect on the production of TNF-α.

### Advantage of the invention

The imidazol derivatives represented by the general formula(I) inhibit the production of IL-4 from mast cells and the production of TNF-α from macrophages. Compounds of the present invention therefore are effective for treating diseases in which IL-4 and/or TNF-α are involved but more particularly are effective in the treatment of auto-immunodiseases such as for example rheumatoid arthritis, multiple sclerosis, lupus erythematodes discoïdalis, systemic lupus erythematodes but also psoriasis.

In addition, the inhibitory efficacy of imidazol derivatives of general formula (I) in term of IL-4 and its cell dependent inhibitory activity in term of TNF-α production is a unique combination of anti-cytokine activity. The moderate and cell dependent reduction of TNF-α production by imidazol derivatives of general formula (I) presents an advantage in not reducing the overall immune system reactivity as it is seen with e.g. corticosteroids.

### Figures

Fig. 1 Effects of mizolastine, terfenadine, ketotifen and dexamethasone on antigen-induced IL-4 production from mouse BMMC. Drugs were added 60 minutes before antigen challenge. Each bar represents the mean ± S.E. (n=4, except blank n=3). Significant differences from control were calculated by Dunnett multiple comparison procedure (**, p<0.01)
Fig. 2 Effects of mizolastine, terfenadine, ketotifen and dexamethasone on antigen induced TNF-α production from mouse BMMC. Drugs were added 30 minutes before antigen challenge. Each bar represents the mean ± S.E. (n=3∼4). Significant differences from control were calculated by Dunnett multiple comparison procedure (**; p<0.01).
Fig. 3 Effects of mizolastine and terfenadine on okadaic acid-induced TNF-α production from mouse peritoneal macrophages. Drugs were added 30 minutes before the stimulation. Each bar represents the mean ± S.E. (n=3, except blank n=2). Significant differences from control were calculated by Dunnett multiple comparison procedure (**; p<0.01).

## Claims

1. Use of a compound represented by the following general formula (I) 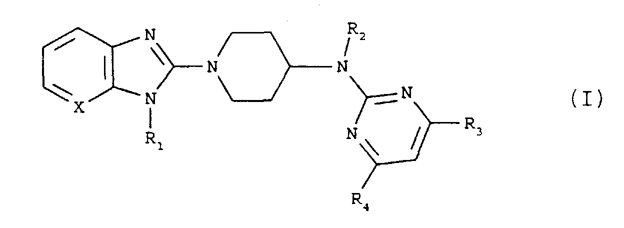 where X is a group -CH- or a nitrogen atom ;
R₁ is a hydrogen atom, a non-substituted benzyl group, a benzyl group substituted by up to three substitutional groups selected from the group consisting of halogen, trifluoromethyl, (C₁₋₄) alkyl, (C₁₋₄) alkoxy, cyano, methylthio, methylsulfinyl or methylsulfonyl groups ; a non-substituted heterocyclic methyl group wherein the heterocyclic part is a pyridyl, thienyl or furyl ; or a heterocyclic methyl group wherein the heterocyclic part is substituted by one or more substitutional groups selected from the group consisting of halogen, trifluoromethyl, (C₁₋₄) alkyl, (C₁₋₄) alkoxy, cyano, methylthio, methylsulfinyl or methylsulfonyl groups ;
R₂ is a hydrogen atom or (C₁₋₄) alkyl group ;
R₃ is a hydrogen atom or hydroxy group ;
R₄ is a hydrogen atom or (C₁₋₄) alkyl group, its tautomer when R₃ is an hydroxy group, their pharmaceutically permissible acid addition salts, or any of their hydrates for the manufacture of a medicament for creating an auto-immunodisease.

2. Use of a compound according to claim 1 wherein said compound is 2-([1-{1-[(4-fluorophenyl)methyl]-1*H*-benzimidazol-2-yl}-4-piperidyl)methylamino)-4-pyrimidinol.

3. Use of a compound according to claim 1 or 2 wherein said auto-immunodisease is rheumatoides arthritis, multiple sclerosis, lupus erythematodes discoïdalis and systemic lupus erythematodes.

4. Use of a compound according to claim 1 or 2 for the manufacture of a medicament for treating psoriasis.

5. Use of a compound according to claim 1 or 2 for the manufacture of a medicament for treating diseases in which IL-4 and / or TNF-α are involved.
